# EUROPEAN PATENT SPECIFICATION

(11) **EP 2 119 471 B1**
(45) Date of publication and mention of the grant of the patent: **17.08.2011**
(21) Application number: 09006452.8
(22) Date of filing: 13.05.2009
(51) Int. Cl.: A61N 1/05, A61N 1/39

(54) **High-voltage tolerant multiplex multi-electrode stimulation systems**
Hochspannungstolerante Multiplexstimulationssysteme mit mehreren Elektroden
Systèmes de stimulation à électrodes multiples à multiplexage tolérant aux hautes tensions

(30) Priority: 13.05.2008 US 52976 P
(43) Date of publication of application: 18.11.2009
(73) Proprietor: Proteus Biomedical, Inc., Redwood City, CA 94065 (US)
(72) Inventor: Zdeblick, Mark, Portolla Valley, California 94028 (US)
(74) Representative: Duxbury, Stephen

(56) References cited:
- US-A- 5 325 870
- US-A- 5 999 849
- US-A1- 2006 058 588

## Description

### INTRODUCTION

Implantable defibrillators have proven to be lifesaving for many heart patients. Implantable defibrillators represent a considerable improvement over external defibrillators, which depend on close physical proximity to the patient suffering from a cardiac event. In addition, implantable defibrillators do not require a skilled observer to be present for a life-saving electrical stimulus to be administered.

Current implantable defibrillators typically require a coil in the right atrium of the heart. This coil is used to provide electrical stimulation which defibrillates the heart in a desirable manner.

However, with the currently available devices, a very large voltage at very high power is required for therapeutic efficacy. For instance, during an electrical stimulation event 10, 20, or even 30 Joules may be released into the coil and returned by the can. This exceptionally high amount of power is needed to capture the tissue of all of the heart between the single location of the coil and the can.

### SUMMARY

High-voltage tolerant multiplex multi-electrode stimulations systems are provided. Aspects of the systems include a multiplex multi-electrode stimulation device, such as lead, configured to deliver high-voltage stimulation pulses through low-voltage satellites. Also provided are low-power implantable defibrillation systems, where such systems may include a high-voltage tolerant multiplex multi-electrode stimulation system.

US 2006 /0058588 discloses methods and apparatus for tissue activation and monitoring.

### BRIEF DESCRIPTION OF THE FIGURES

FIGS. 1A and 1B provide views of an implantable system according to the invention in which the left-ventricular multiplex multi-electrode stimulation system is a multiplex multi-electrode lead.
FIGS. 2A and 2B provide views of a satellite lead according to an embodiment of the invention before and after configuration.
FIG. 3 provides a view of a left-ventricular multiplex multi-electrode stimulation system that comprises an epicardial mesh.
FIG. 4 provides a graph showing voltage applied to a configured lead according to an embodiment of the invention.

### DETAILED DESCRIPTION

A high-voltage tolerant multiplex multi-electrode stimulations system according to claim 1 is provided. Aspects of the systems include a multiplex multi-electrode stimulation device, such as lead, configured to deliver high-voltage stimulation pulses through low-voltage satellites. Also provided are low-power implantable defibrillation systems, where such systems may include a high-voltage tolerant multiplex multi-electrode stimulation system.

Aspects of the invention include high-voltage tolerant multiplex multi-electrode stimulation systems. These systems include a multiplex multi electrode stimulation device comprising two or more low-voltage satellites. Stimulation systems of interest include lead and mesh systems, as described in greater detail below. Each of the low-voltage satellites comprises at least a first electrode, a second electrode and an integrated circuit. As the satellites are low-voltage satellites, they include one or more low-voltage components, such as components designed to accept voltages of 10V or less, including 5V or less. Such low-voltage satellites may not, when not operated in accordance with the system of the present invention, function property when voltages exceeding these values are applied to them.

Systems of these embodiments of the invention further include a control unit comprising a processor configured to deliver a high-voltage electrical stimulus through the multiplex multi-electrode stimulation device without damaging the circuitry for the low-voltage satellites. Because of the configuration of these systems, the systems may deliver an electrical stimulus that exceeds the voltage for which the low-voltage satellites are configured to handle by a factor of 2 or more, such as 5 or more, including 10 or more. Systems of the invention are configured to maintain a differential voltage of 4V or less across the integrated circuits of the low-voltage satellites when an electrical stimulus of 100V is delivered through the stimulation device, without damaging the circuitry of the integrated circuits.

In systems of the invention, the two or more low-voltage satellites are electrically coupled to first and second conduction elements. The processor of the control unit is configured to couple the first electrode of a given satellite to the first conduction element and the second electrode of the same satellite to the second conduction element. In addition, the processor is configured to electrically short the first conduction element to the second conduction element and then deliver an electrical stimulus via the first and second conduction elements.

Via the high voltage tolerant multiplex multielectrode stimulation systems, electrical stimulation may be delivery to a variety of different types of tissue, which include, but are not limited to, muscle tissue (such as cardiac tissue), neural tissue, gastro-intestinal tissue, organ tissue, etc. Examples of applications in which systems of the invention may find use include stimulation applications, ablation applications, etc.

One type of stimulation application of interest is cardiac defibrillation. For ease of description only, additional aspects of the systems of the invention are further described in terms of low power cardiac defibrillation applications. It should also be noted that in the following discussion, the systems employed in the below described low-power cardiac defibrillation systems are not limited to using high-voltage tolerant multiplex multi-electrode stimulation systems as described.

Aspects of the low-power implantable defibrillation systems of the invention include a right-side electrical stimulation element, a left-ventricular multiplex multi-electrode stimulation system and a control unit. The control unit comprises a processor configured to deliver electrical energy through the right-side electrical stimulation element and the left-ventricular multiplex multi-electrode stimulation system in a manner sufficient to defibrillate a heart.

Low-power cardiac defibrillation implantable systems of interest include a right-side electrical stimulation element. By right-side electrical stimulation element is meant a structure or device that is configured to deliver electrical energy to a right-side region of a heart. By right-side region is meant an interior or exterior region located on the right side of a heart. Right-side regions of interest include both right-atrium and right-ventricular regions, where the region may be inside of the heart, such as in the right atrium or the right ventricle, or on the outside of the heart proximal to these locations. Right-side regions also include regions proximal to these regions of the heart.

The right-side electrical stimulation element may vary. In some instances, the right-side electrical stimulation element is made up of one or more leads. The one or more leads may each include one or more distally located electrodes that are electrically coupled to a proximal end connector, such as DS-1 connector configured to connect to an implantable control unit, such as the implantable control units described in greater detail below. The one or more electrodes may have any convenient configuration, where in some instances the one or more electrodes have a coil configuration. Of interest are single chamber systems that may include a single lead configured to be placed in the right ventricle (for example to treat ventricular arrhythmia). Also of interest are dual chamber systems that may include one lead placed in the right ventricle and a second lead placed in the right atrium. In both single and dual chamber systems, a high voltage coil may be located on the lead or leads for delivering a cardioversion/defibrillation shock. Some leads may carry two or more coils, as desired. Additional electrodes may also be present, e.g., to provide for sensing and/or pacing. Also of interest are systems where the one or more electrodes are outside of but proximal to the right side chambers of the heart. Examples of such systems are systems that include one or more electrodes positioned intravenously near the heart and proximal to these regions.

Systems of interest further include a left-ventricular multiplex multi-electrode stimulation system. By left-ventricular multiplex multi-electrode stimulation system is meant a structure or device that is configured to deliver electrical energy to the left-ventricular region of a heart. By left-ventricular region is meant an interior or exterior region of the left ventricle of a heart.

As the left-ventricular electrical stimulation system is multiplex, it includes two or more effectors, such as electrodes, that are each coupled to a common conductive member or members, such as a common wire or set of wires. Multiplex systems of interest include those that comprise 3 or less common wires, such as only 2 common wires or only 1 common wire. Multiplex lead structures of interest include those described in U.S. Patent No. 7,214,189 and U.S. patent application serial no. 10/734,490 published as 20040193021

Left-ventricular multiplex multi-electrode stimulation systems of interest include multiplex multi-electrode leads that are configured to be positioned inside of a left ventricle or in a left-ventricular vein. Multiplex multi-electrode leads of interest are leads that include two or more individually addressable electrodes electrically coupled to the same wire or wires. While the electrodes of the lead may vary, the individually addressable electrodes are present on satellites. Leads of interest include two or more satellites, such as three or more, four or more, five or more, ten or more, fifteen or more, twenty or more, etc. satellites, as desired. Satellites are structures that include at least a first electrode, a second electrode and an integrated circuit. In satellites of interest, the first and second electrodes are individually addressable, such that the satellites include two or more different individually addressable electrodes that are coupled to the same integrated circuit, which may be viewed as the satellite controller and may also be referred to herein as a "chip". The multiplex multi-electrode lead comprises first and second conduction elements, as well as two or more satellites electrically coupled to the conduction elements.

Satellites may have a variety of different configurations. Of interest are satellites that have a segmented structure. Segmented structures of interest include, but are not limited to, those described in U.S. Patent No. 7,214.189 and United States Patent Application Serial Nos. 11/793,904 published as 20080255647 and 11/794,016 published as 20080312726

Left-ventricular multiplex multi-electrode stimulation systems of interest also include deployable epicardial multi-electrode stimulation systems that include a deployable epicardial mesh, such as those described in PCT application serial no. PCT/US2006/025648 arid published as WO 2007/005641.

Systems of the invention further include an implantable control unit. The implantable control unit is an implantable device that includes a processor configured to deliver electrical energy through the right-side electrical stimulation element and the left-ventricular multiplex multi-electrode stimulation system in a manner sufficient to defibrillate a heart. Control units of interest include a housing which is often referred to in the art as the "can", "case" or "case electrode". The housing is any structure that provides for an environment for the processor that is protected from the implanted environment of the body. In addition to providing this functionality, the housing may serve as a return electrode in some instances, such as where unipolar leads are employed. In such instances, the processor may be programmed to use the housing as the return electrode. In such instances, the housing may be used as a return electrode alone or in combination with one or more additional lead electrodes, such as the coil electrodes, for generating electrical shock. The housing may further include one or more connectors, e.g., for operably connecting to one or more stimulation elements, such as the leads as described above.

The processor of the control unit may be embodiments in any convenient fashion, such as in the form of a programmable microcontroller which controls the various modes of operation of the system. The microcontroller may include a microprocessor, or equivalent control circuitry, designed specifically for controlling the delivery of stimulation therapy and may further include RAM or ROM memory, logic and timing circuitry, state machine circuitry, and I/O circuitry. The microcontroller may include the ability to process or monitor input signals (data) as controlled by a program code stored in a designated block of memory.

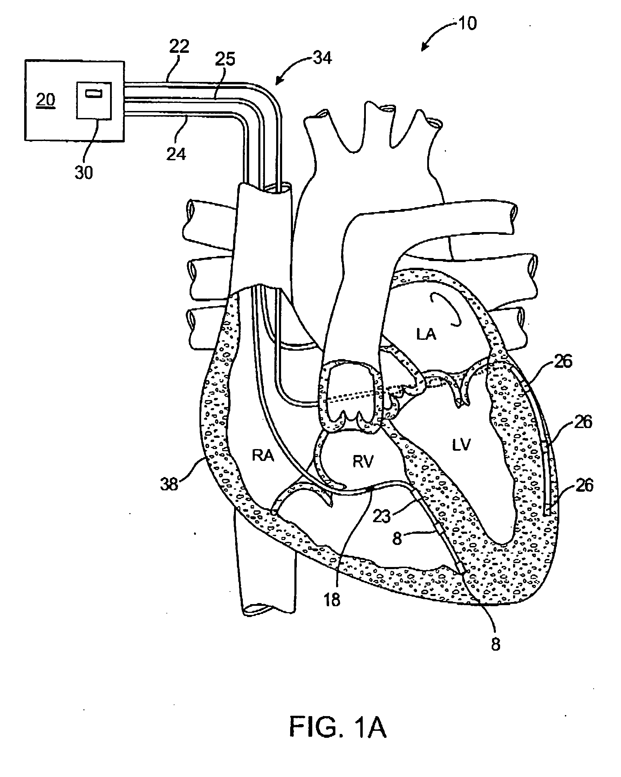

An example of a system of interest is shown in FIG. 1A. FIG. 1A illustrates system 10 implanted in a heart. System 10 includes control unit 20 that includes processor 30. Also shown is the right-side electrical stimulation element made up of a right-atria lead 25 and right-ventricle lead 24. At least one of the right-atria and right-ventricle leads 25 and 24 includes a coil electrode for delivering a defibrillation electrical stimulation to the heart during use. As shown in FIG. 1A, right-ventricle lead 24 includes coil electrode 23 as well as sense/pacing electrodes 8. Also shown is pressure sensor 18, which is optional.

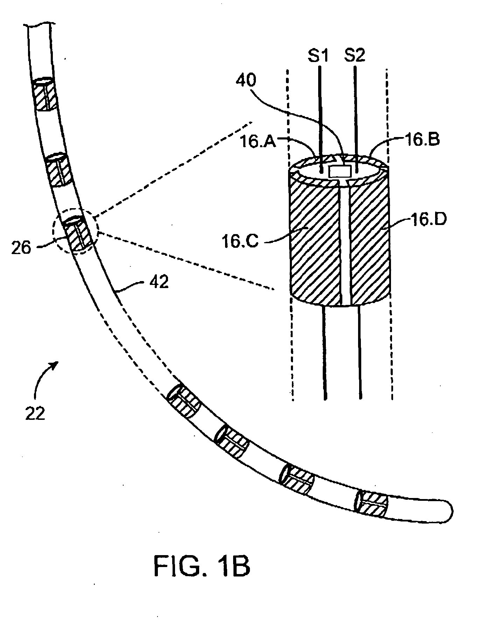

In the system shown in FIG. 1A, the left-ventricular multiplex multi-electrode stimulation system 34 includes lead 22, which includes three different satellites 26. For convenience, not all of the satellites on lead 22 are shown in FIG. 1A. FIG. 1B provides a more detailed view of the lead 22. As seen in FIG. 1B, lead 22 includes multiple satellites 26 present on lead body 42. Each satellite includes four segmented electrodes 16A to 16D positioned about integrated circuit controller 40. Integrated circuit controller 40 is coupled to S1 and S2, which are common conductors to which all satellites of the lead are electrically coupled.

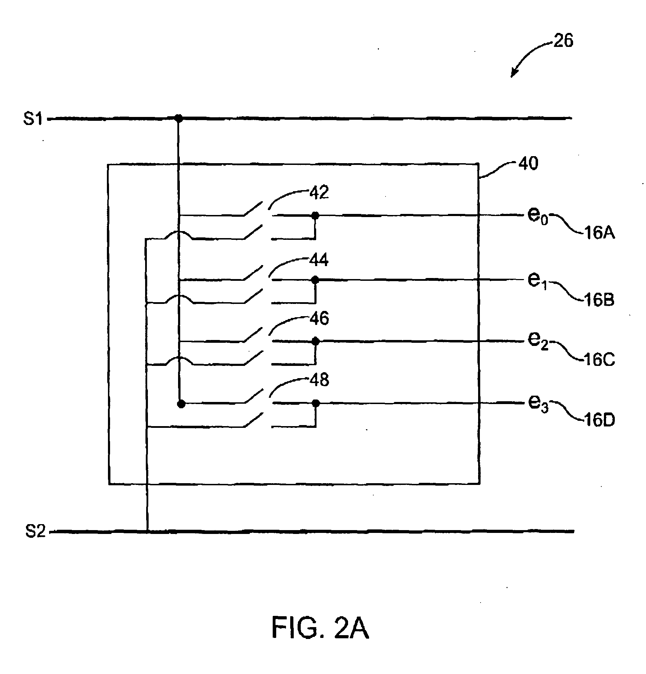

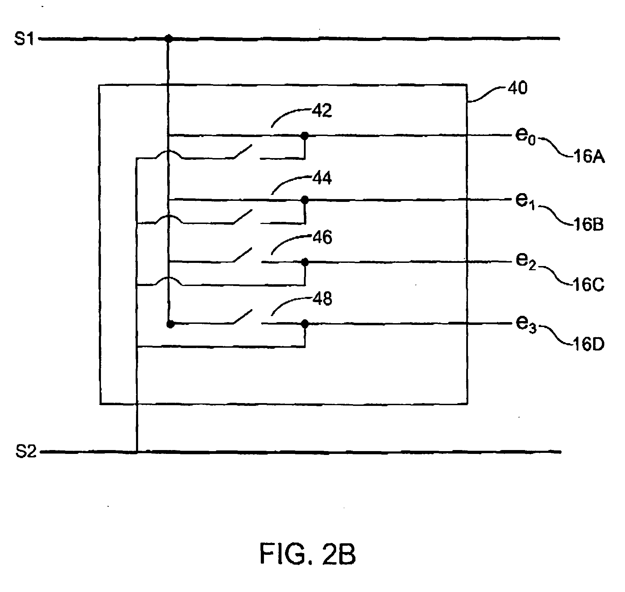

As summarized above, the processor of the control unit is configured to deliver electrical energy through the right-side electrical stimulation element and the left-ventricular multiplex multi-etectrode stimulation system in a manger sufficient to defibrillate a heart. In one embodiment of operation of the system in which the left-ventricular multiplex multi-electrode stimulation system is a left-ventricular multiplex multi-electrode lead, the processor first configures at least the left-ventricular multiplex multi-electrode lead to produce a configured lead. This configuration step may include sending out a sequence of programming commands in such a way that half of the electrodes on each satellite are connected to S1 and the other half are connected to S2. This configuration step is illustrated in FIGS. 2A and 2B. FIG. 2A shows satellite 26 of FIG. 1B prior to configuration. In FIG. 2A, satellite 26 includes integrated circuit controller 40. Integrated circuit controller 40 includes switches 42, 44, 46 and 48 coupled to electrodes 16A (e₀), 16B (e₁), 16C (e₂) and 16D (e₃), respectively. In FIG. 2A, each of switches 42, 44, 46 and 48 are shown in the open position, such that none of the electrodes 16A, 16B, 16C and 16D are coupled to S1 and S2. In FIG. 2B. switches 42 and 44 are configured such that electrodes 16A and 16B are coupled to S1, while switches 46 and 48 are configured such that electrodes 16C and 16D are coupled to S2. Accordingly, integrated circuit controller 40 is configured to couple a first electrode of a satellite to a first conduction element and a second electrode of the same satellite to a second conduction element to produce a configured lead. This configuration may be checked with either multiple "switch commands" or a single "defibrillation configuration" command verifying this unique configuration.

After the programming of the satellites is completed and a configured lead is obtained, S1 may be electrically shorted to S2. In these instances, the processor is configured to electrically short the first conduction element to the second conduction element of the configured lead. Following this step, the control unit may then deliver an electrical stimulus via the first and second conduction elements, for example where the electrical stimulus is distributed evenly between the first and second conduction elements. The effect of this arrangement is to distribute the therapeutic current between the various wires generally and between the two LV wires, S1 and S2, evenly.

Where desired, the electrically tied S1 and S2 conduction elements may be further electrically tied to the right side electrical stimulation element, e.g., by shorting S1 and S2 collectively to the wire connected to the defibrillator coil placed in the right atrium and/or right ventricle. In this embodiment, before the defibrillation therapy occurs, S1 and S2 are tied together electrically. In this configuration, S1 and S2 are held at the same electrical potential as the electrode of the right side electrical stimulation element, e.g., a RV coil. As a result, the three wires, that is the RV coil, S1 and S2, are all electrically connected to each other. That common potential is then varied to deliver the defibrillation current and voltage. The three wires, S1, S2, and the wire going to the coil, all go up and down together to deliver the defibrillation pulse. As such, the processor may be configured to electrically short the first and second conduction elements of the lead to the right side electrical stimulation element.

Alternatively, S1 and S2 may be electrically shorted to each other and the right-ventricular coil may be controlled independently, such that two different defibrillation voltages may then be used either simultaneously or sequentially. In this embodiment, S1 and S2 are electrically tied together, but the right-ventricular coil is independent of S1 and S2. In this embodiment, it is possible for S1 and S2 to be at a different potential other than the right-ventricular voltage at any point in time. For example, voltage pulses may be placed simultaneously on S1 and S2 and the right-ventricular coil lead, but at different potentials. Or, as another example, the voltage placed on S1 and S2 may come before or after the pulse placed on the right-ventricutar coil, or some combination of both. Accordingly, the processor may be configured to independently deliver an electrical stimulus to the right-side electrical stimulation element (for example the right-ventricular lead) and the configured lead that includes S1 and S2.

In the system shown in FIGS. 1A and 1B, an important therapeutic application for the system is the use of the left-ventricular lead for pacing out of a pathological arrhythmia state. Pacing pulses along the left-ventricular lead and administering these pulses at a low voltage allows the programmer to pace the heart out of defibrillation. Different ways of applying pulses through the left-ventricular lead take the heart out of a fibrillation event. Systems of the invention find use in reactivating the beating of even a non-active heart or in stopping the fibrillations of just the atria, just the ventricles, or both.

During use, the systems have a specific electrical configuration. In a first embodiment for providing defibrillation therapy, the circuits are all on. Specifically, the four switches to the four electrodes are configured so that two are connected to S2, and two are connected to S1, e.g., as shown in FIG. 2B This configuration would be the same for all the satellite electrodes on the leads as shown in FIGS. 1A and 1B.

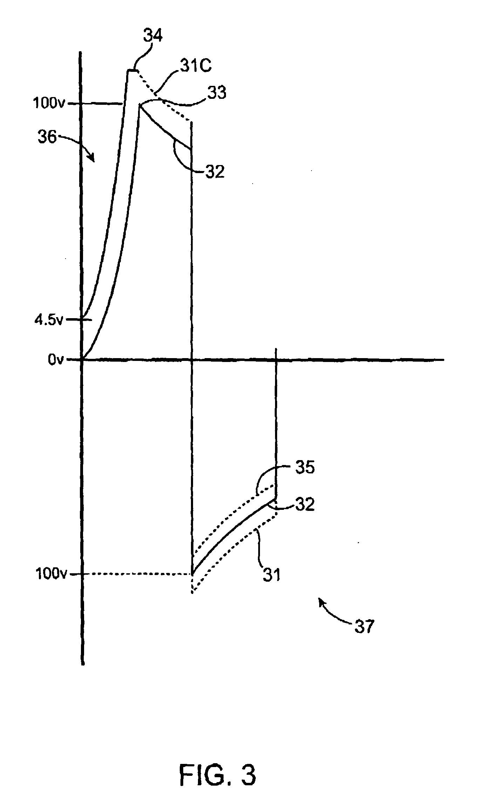

Following configuration, such as described above, the control unit delivers an electrical stimulation pulse to cardiac tissue, e.g., to defibrillate a heart. One embodiment of delivery of electrical energy by a system of the invention is depicted graphically in FIG 3. The programming voltage of these switches is held by what may be termed Vhigh 31, which is typically about 4.5 V above the voltage on S1. As soon as S1 and S2 are connected to each other, then Vhigh 31 will naturally be at about 4.5 volts above that level. As S1 and S2 rise to even 100 volts, Vhigh 31 stays at a constant 4.5 V above S1. As shown in FIG. 3, with first pulse 36, Vhigh 31 starts at between 4 and 5 volts. S1 and S2 are shown as solid line 32 with Vhigh 31 displayed as a dotted line. As shown in FIG. 3, S1 and S2 (32) start out at zero. As the therapeutic stimulus is rendered, these voltage levels can go up to a very large value. For purposes of this example, this level is 100 volts for S1 and S2 (32), as illustrated at point 33 of the graph.

Vhigh would rise to whatever that voltage is, and 4.5 more (as illustrated by point 34 in the graph). The differential voltage across the integrated circuit is never more than about 4 volts because S1 and S2 are connected to each other and all of the electrodes are electrically tied to either S1 or S2. As described previously, in this embodiment, in addition to the substrate, there are only six external connections to the integrated circuit. One skilled in the art may appreciate the number of electrodes per integrated circuit may be increased to many more electrodes per satellite or fewer without changing this protection to the circuitry inherent in this design, provided that all of the electrodes are connected to either S1 or S2. Even if most of the electrodes are connected to S1 or S2, the system will operate safely due to the distribution of voltage around the satellite by the surrounding blood and tissue. In some instances, all switches are connected to either S1 or S2. In general, the voltage applied to S1, S2 and the right ventricular coil is with respect to the can and not with respect to one wire vs. the other wire. However, in some instances, a voltage is applied between the left-ventricular lead and the right-ventricular coil.

Regardless of the configuration, after the first defibrillation pulse 36 is delivered, a second pulse 37 of equal magnitude but opposite polarity may be delivered, as desired. In this case, the Vhigh 31 is again 4.5 volts above the negative voltage 32. The Vlow 35 (substrate voltage) would be slightly less than that. In all cases the differences in voltages across the integrated circuit never exceed 5 volts. In this way, the system provides a bipolar 100 volt pacing pulse through the integrated circuit and into the tissue without causing even the slightest risk of damaging the electronic circuits. After this combination of pulses (which are typically referred to as bipolar defibrillation pulses) is delivered, the resulting voltage across each electrode decays to zero.

While the above describes aspects of one type of stimulation protocol that may be implemented by systems of the invention, any convenient stimulation protocol may be employed.

Advantages of the systems are numerous. By distributing the current geographically across the other side of the heart, the power consumption can be reduced by 20% or more, such as 40% or more, 50% or more, etc. As such, systems of the invention allow for a dramatic reduction in the size of the battery or an increase in the lifetime of the battery, both of which are important advantages to the patient.

There are many biological advantages of having lower effective defibrillation therapy power requirements. Providing effective therapy at lower voltages lowers the risk of burning the tissue at the therapy site. Reducing the voltage by half significantly reduces this injury.

There is also an advantage to putting more satellites on the lead to accomplish this function. A simple embodiment of the system can have just four satellites. However, it is advantageous is some applications to have ten satellites distributed through the cardiac vein around the other aspects of the heart. In other applications, it is advantageous to put additional multiplexed electrodes on the right-ventricular leads to essentially make all the leads that go into the heart a virtual defibrillation coil. When needed, all of these multiplexed electrodes could be connected to a wire that provides defibrillation energy, and this therapeutic electrical energy would be distributed throughout the heart. In addition, these multiple leads may each be capable of delivering a defibrillation pulse and may each be fault tolerant, such that a failure in one satellite, one electrode or one lead would not cause a failure of the system. As such, an effective defibrillation would be delivered through the still-functional wires, satellites and electrodes. This configuration is a very effective, high reliability configuration that lowers the effective therapeutic power while still accomplishing the defibrillation of the heart.

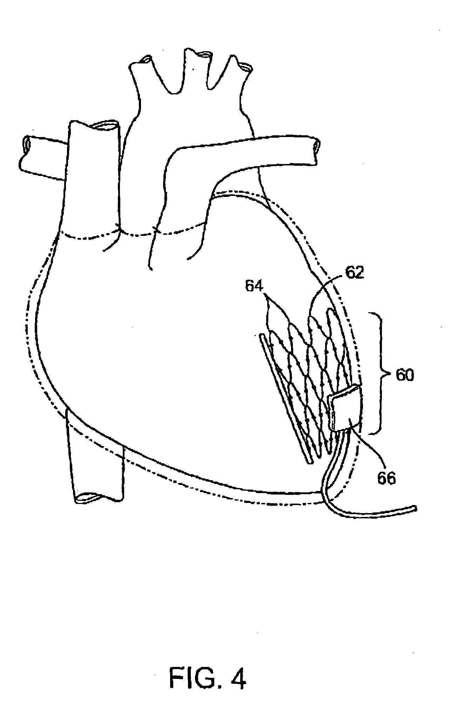

As indicated above, in some instances the systems include a deployable epicardial mesh device, e.g., as described in PCT application serial no. PCT/US2006/025648 and published as WO 2007/005641. This application describes a mechanical mesh that incorporates a single conductor which goes on the outside of the heart. An example of an embodiment of this type of left ventricular multiplex multi-electrode stimulation system is shown in FIG. 4. In FIG. 4, epicardial mesh 60 includes mesh support element 62 and a plurality of satellites 64. Also shown is attachment element 66, which is a balloon attachment element 66 that can be used to stabilize the mesh 60 against the heart during minimally invasive fixation with sutures, staples, or electrically active microhooks, as desired. Satellites 64 are analogous to satellites 26 shown in FIG. 2B. As described above, further details of such systems are provided in PCT application serial no. PCT/US2006/025648, published as WO 2007/005641. In this embodiment of the present system, several approaches are available. Cardiac resynchronization therapy can be implemented by selectively turning on one or more electrodes at any point of the mesh. Motion sensing may be sensed at any point by selectively turning on any electrode connected to the mesh, which is insulated from the cardiac tissue. Alternatively, all of the electrodes connected to the mesh may be turned on. In this mode, the defibrillation mode, the level of therapeutically effective voltage may be reduced to the order of 10 volts or less, since the energy is completely distributed around the heart in a uniform manner. A very small voltage may be effective as the device approaches 100 electrodes on the outside of the heart.

Advantages of the various system embodiments described herein are numerous. Embodiments of systems of the invention make use of a left-ventricular multiplex mutti-electrode stimulation system (for example a lead) in such a way as to distribute the defibrillation current to both the coil on the right side and the electrodes on the left side. This unique capability distributes the power of the electrical stimulus more efficiently. As such, systems of the invention provide for heart defibrillation at substantially lower power levels than what are possible using reference devices that employ only a coil in the right atrium or ventricle. In comparison to such reference devices, the power levels employed by systems of the invention are 15 J or less, such as 10 J or less, including 5 J or less. In some instances, the voltage of a given therapeutic electrical stimulation may be 1 kV or less, such as 100 V or less, including 20 V or less.

With systems of the invention, the size of the control unit can be much smaller than in currently available defibrillation devices, allowing unique surgical placement opportunities, and less medical complications for patients. With some systems of the invention, 100 volt pacing pulses can be provided through an implanted computer chip on a lead and into the cardiac tissue without any risk of damaging the electronic circuits. Power batteries are conserved for later therapy. The tissue damaging side-effects of defibrillation therapy are considerably reduced due to the lower voltages and current densities. The trauma of a large, often unexpected, shock to the patient is much ameliorated. Many other advantages are also accrued by the low power implanted defibrillator.

In addition, systems of the invention may be employed for electrical stimulation therapies other than defibrillation. For example, systems of the invention may be employed for cardiac resynchronization therapy (CRT) when programmed differently. In another mode, the system may be re-programmed to stimulate at a single location for CRT, which may consume less power and produces a better therapeutic response. Accordingly, the processor of the systems may be further configured to deliver energy to the left-ventricular multiplex multi-electrode stimulation system in a manner sufficient to perform CRT.

Systems of the invention may be employed with a variety of different subjects. Generally such subjects are "mammals" or "mammalian," where these terms are used broadly to describe organisms which are within the class mammalia, including the orders carnivore (e.g., dogs and cats), rodentia (e.g., mice, guinea pigs, and rats), and primates (e.g., humans, chimpanzees, and monkeys). In certain embodiments, the subjects will be humans.

It is to be understood that this invention is not limited to particular embodiments described, as such may vary. It is also to be understood that the terminology used herein is for the purpose of describing particular embodiments only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

## Claims

1. A multiplex multi-electrode stimulation system comprising:
a multiplex multi-electrode stimulation device (10) comprising two or more low-voltage satellites (26); and
a control unit (20) comprising a processor (30) configured to deliver a high-voltage electrical stimulus through the multiplex multi-electrode stimulation device,
wherein each of the low-voltage satellites (26) comprises at least a first electrode (16A-16D), a second electrode (16A-16D) and an integrated circuit,
wherein the system (10) is configured to maintain a differential voltage of 4V or less across the integrated circuit when an electrical stimulus of 100V is delivered through the stimulation device,
wherein the multiplex multi-electrode stimulation system comprises first and second conduction elements (51, 52) and the two or more low-voltage satellites (26) are electrically coupled to the first and second conduction elements (51, 52),
wherein the processor (30) is configured to couple the first electrode of a satellite to the first conduction element and the second electrode of the same satellite to the second conduction element, **characterized in that**
the processor (30) is configured to electrically short the first conduction element to the second conduction element and then deliver an electrical stimulus via the first and second conduction elements (51, 52).

2. An implantable system (10) for defibrillating a heart, the system comprising:
(a) a right-side electrical stimulation element (24, 25);
(b) a left-ventricular multiplex multi-electrode stimulation system (34); and
(c) a control unit (20) comprising a processor (30) configured to deliver electrical energy through the right-side electrical stimulation element (24, 25) and the left-ventricular multiplex multi-electrode stimulation system (34) in a manner sufficient to defibrillate a heart,
wherein the left ventricular multiplex multi-electrode stimulation system (34) is a multiplex multi-electrode lead (22), wherein the multiplex multi-electrode lead comprises first and second conduction elements and two or more satellites (26) electrically coupled to the first and second conduction elements (51, 52),
wherein each satellite (26) comprises at least a first electrode (16A-16D), a second electrode (16A16D) and an integrated circuit,
wherein the processor (30) is configured to couple the first electrode of a satellite to the first conduction element and the second electrode of the same satellite to the second conduction element to produce a configured lead, **characterized in that**
the processor is configured to electrically short the first conduction element to the second conduction element of the configured lead and then deliver an electrical stimulus via the first and second conduction elements (51, 52).

3. The system of claim 2, wherein the processor (30) is configured to electrically short the first and second conduction elements of the lead to the right-side electrical stimulation element (24, 25).

4. The system of claim 2, wherein the processor (30) is configured to independently deliver an electrical stimulus to the right-side electrical stimulation element (24, 25) and the configured lead.

5. The system according to Claim 2, wherein the processor (30) is further configured to deliver energy to the left-ventricular multiplex multi-electrode stimulation system (34) in a manner sufficient to perform cardiac resynchronization therapy or wherein the left-ventricular multiplex multi-electrode stimulation system (34) comprises an epicardial mesh (60).

6. Assembly comprising a multiplex multi-electrode stimulation system according to claim 1, and an implantable system for defibrillating a heart according to any of the claims 2-5.

7. Use of a multiplex multi-electrode stimulation system according to claim 1 and/ or an implantable system according to any of the claims 2-5 for producing an assembly according to claim 6.

## Patentansprüche

1. Multiplex-Mehrelektroden-Stimulationssystem, Folgendes umfassend:
eine Multiplex-Mehrelektroden-Stimulationsvorrichtung (10), die zwei oder mehr Niederspannungssatelliten (26) umfasst; und
eine Steuerungseinheit (20), die einen Prozessor (30) umfasst, der dafür eingerichtet ist, über die Multiplex-Mehrelektroden-Stimulationsvorrichtung einen elektrischen Hochspannungs-Anregungsimpuls zu liefern,
wobei jeder der Niederspannungssatelliten (26) wenigstens eine erste Elektrode (16A-16D), eine zweite Elektrode (16A-16D) und eine integrierte Schaltung umfasst,
wobei das System (10) dafür eingerichtet ist, über die integrierte Schaltung eine Spannungsdifferenz von 4 V oder weniger aufrechtzuerhalten, wenn durch die Stimulationsvorrichtung ein elektrischer Anregungsimpuls von 100 V geliefert wird,
wobei das Multiplex-Mehrelektroden-Stimulationssystem ein erstes und zweites Leitungselement (51, 52) umfasst und die zwei oder mehr Niederspannungssatelliten (26) elektrisch mit dem ersten und zweiten Leitungselement (51, 52) verbunden sind,
wobei der Prozessor (30) dafür eingerichtet ist, die erste Elektrode eines Satelliten mit dem ersten Leitungselement und die zweite Elektrode des selben Satelliten mit dem zweiten Leitungselement zu verbinden,
**dadurch gekennzeichnet, dass**
der Prozessor (30) dafür eingerichtet ist, das erste Leitungselement mit dem zweiten Leitungselement elektrisch kurzzuschließen und dann über das erste und zweite Leitungselement (51, 52) einen elektrischen Anregungsimpuls zu liefern.

2. Implantierbares System (10) zur Defibrillation eines Herzen, wobei das System Folgendes umfasst:
(a) ein elektrisches Stimulationselement (24, 25) der rechten Seite,
(b) ein Multiplex-Mehrelektroden-Stimulationssystem (34) des linken Ventrikels; und
(c) eine Steuerungseinheit (20), die einen Prozessor (30) umfasst, der dafür eingerichtet ist, über das elektrische Stimulationselement (24, 25) der rechten Seite und das Multiplex-Mehrelektroden-Stimulationssystem (34) des linken Ventrikels elektrische Energie zu liefern, und dies in einer Weise, die ausreichend ist, um ein Herz zu defibrillieren,
wobei das Multiplex-Mehrelektroden-Stimulationssystem (34) des linken Ventrikels eine Multiplex-Mehrelektrodenleitung (22) ist und wobei die Multiplex-Mehrelektrodenleitung ein erstes und zweites Leitungselement und zwei oder mehr Satelliten (26) umfasst, die mit dem ersten und zweiten Leitungselement (51, 52) elektrisch verbunden sind,
wobei jeder Satellit (26) wenigstens eine erste Elektrode (16A-16D), eine zweite Elektrode (16A-16D) und eine integrierte Schaltung umfasst,
wobei der Prozessor (30) dafür eingerichtet ist, die erste Elektrode eines Satelliten mit dem ersten Leitungselement und die zweite Elektrode des selben Satelliten mit dem zweiten Leitungselement zu verbinden, um eine konfigurierte Leitung herzustellen,
**dadurch gekennzeichnet, dass**
der Prozessor dafür eingerichtet ist, das erste Leitungselement mit dem zweiten Leitungselement der konfigurierten Leitung elektrisch kurzzuschließen und dann über das erste und zweite Leitungselement (51, 52) einen elektrischen Anregungsimpuls zu liefern.

3. System nach Anspruch 2, wobei der Prozessor (30) dafür eingerichtet ist, das erste und zweite Leitungselement der Leitung mit dem elektrischen Stimulationselement (24, 25) der rechten Seite elektrisch kurzzuschließen.

4. System nach Anspruch 2, wobei der Prozessor (30) dafür eingerichtet ist, unabhängig einen elektrischen Anregungsimpuls an das elektrische Stimulationselement (24, 25) der rechten Seite und die konfigurierte Leitung zu liefern.

5. System nach Anspruch 2, wobei der Prozessor (30) außerdem dafür eingerichtet ist, Energie an das Multiplex-Mehrelektroden-Stimulationssystem (34) des linken Ventrikels in einer Weise zu liefern, die ausreichend ist, um eine kardiale Resynchronisationstherapie durchzuführen, oder wobei das Multiplex-Mehrelektroden-Stimulationssystem (34) des linken Ventrikels ein epikardiales Netzwerk (60) umfasst.

6. Aufbau, der ein Multiplex-Mehrelektroden-Stimulationssystem nach Anspruch 1 und ein implantierbares System zur Defibrillation eines Herzen nach einem der Ansprüche 2 bis 5 umfasst.

7. Verwendung eines Multiplex-Mehrelektroden-Stimulationssystem nach Anspruch 1 und/oder eines implantierbaren Systems nach einem der Ansprüche 2 bis 5 zur Herstellung eines Aufbaus nach Anspruch 6.

## Revendications

1. Système de stimulation à électrodes multiples à multiplexage comprenant :
un dispositif de stimulation à électrodes multiples à multiplexage (10) comprenant deux satellites basse tension (26), ou plus ; et
une unité de commande (20) comprenant un processeur (30) configuré pour délivrer un stimulus électrique haute tension par l'intermédiaire du dispositif de stimulation à électrodes multiples à multiplexage,
dans lequel chacun des satellites basse tension (26) comprend au moins une première électrode (16A-16D), une seconde électrode (16A-16D) et un circuit intégré,
dans lequel le système (10) est configuré pour maintenir une tension différentielle de 4 V, ou moins, à travers le circuit intégré lorsqu'un stimulus électrique de 100 V est délivré par l'intermédiaire du dispositif de stimulation,
dans lequel le système de stimulation à électrodes multiples à multiplexage comprend un premier et un second élément de conduction (51, 52) et les deux satellites basse tension (26), ou plus, sont électriquement couplés aux premier et second éléments de conduction (51, 52),
dans lequel le processeur (30) est configuré pour coupler la première électrode d'un satellite au premier élément de conduction et la seconde électrode du même satellite au second élément de conduction,
**caractérisé en ce que** :
le processeur (30) est configuré pour court-circuiter électriquement le premier élément de conduction au second élément de conduction puis délivrer un stimulus électrique par l'intermédiaire des premier et second éléments de conduction (51, 52).

2. Système implantable (10) pour défibriller un coeur, le système comprenant :
(a) un élément de stimulation électrique du côté droit (24, 25) ;
(b) un système de stimulation à électrodes multiples à multiplexage ventriculaire gauche (34) ; et
(c) une unité de commande (20) comprenant un processeur (30) configuré pour délivrer de l'énergie électrique par l'intermédiaire de l'élément de stimulation électrique du côté droit (24, 25) et du système de stimulation à électrodes multiples à multiplexage ventriculaire gauche (34) de manière suffisante pour défibriller un coeur,
dans lequel le système de stimulation à électrodes multiples à multiplexage ventriculaire gauche (34) est un fil conducteur à électrodes multiples à multiplexage (22), où le fil conducteur à électrodes multiples à multiplexage comprend des premier et second éléments de conduction et deux satellites (26), ou plus, couplés électriquement aux premier et second éléments de conduction (51, 52),
dans lequel chaque satellite (26) comprend au moins une première électrode (16A-16D), une seconde électrode (16A-16D) et un circuit intégré,
dans lequel le processeur (30) est configuré pour coupler la première électrode d'un satellite au premier élément de conduction et la seconde électrode du même satellite au second élément de conduction pour produire un fil conducteur configuré,
**caractérisé en ce que** :
le processeur est configuré pour court-circuiter électriquement le premier élément de conduction au second élément de conduction du fil conducteur configuré, puis délivrer un stimulus électrique par l'intermédiaire des premier et second éléments de conduction (51, 52).

3. Système selon la revendication 2, dans lequel le processeur (30) est configuré pour court-circuiter électriquement les premier et second éléments de conduction du fil conducteur vers l'élément de stimulation électrique du côté droit (24, 25).

4. Système selon la revendication 2, dans lequel le processeur (30) est configuré pour délivrer indépendamment un stimulus électrique à l'élément de stimulation électrique du côté droit (24, 25) et au fil conducteur configuré.

5. Système selon la revendication 2, dans lequel le processeur (30) est en outre configuré pour délivrer de l'énergie au système de stimulation à électrodes multiples à multiplexage ventriculaire gauche (34) d'une manière suffisante pour procéder à une thérapie de resynchronisation cardiaque ou dans lequel le système de stimulation à électrodes multiples à multiplexage ventriculaire gauche (34) comprend un maillage épicardique (60).

6. Assemblage comprenant un système de stimulation à électrodes multiples à multiplexage selon la revendication 1 et un système implantable pour défibriller un cour selon l'une quelconque des revendications 2 à 5.

7. Utilisation d'un système de stimulation à électrodes multiples à multiplexage selon la revendication 1 et/ou d'un système implantable selon l'une quelconque des revendications 2 à 5 pour produire un assemblage selon la revendication 6.
